# EUROPÄISCHE PATENTSCHRIFT

(11) **EP 0 493 660 B1**
(45) Veröffentlichungstag und Bekanntmachung des Hinweises auf die Patenterteilung: **03.05.1995**
(21) Anmeldenummer: 91118692.2
(22) Anmeldetag: 02.11.1991
(51) Int. Cl.: A61B 3/06

(54) **Anomaloskop**
Anomaloscope
Anomaloscope

(30) Priorität: 03.01.1991 DE 9100027 U
(43) Veröffentlichungstag der Anmeldung: 08.07.1992
(73) Patentinhaber: Oculus Optikgeräte GmbH, 35582 Wetzlar (DE)
(72) Erfinder: Volkert, Heinz, W-6330 Wetzlar 17 (DE)
(74) Vertreter: Missling, Arne, Dipl.-Ing.

(56) Entgegenhaltungen:
- EP-A- 0 125 459
- DE-A- 3 516 285
- FR-A- 2 520 604
- GB-A- 785 796

## Beschreibung

Die Erfindung betrifft ein Anomaloskop gemäß dem Oberbegriff von Anspruch 1.

Ein derartiges Gerät ist aus der DE-Patentschrift 35 16 285 bereits bekannt. Die Verwendung eines Strahlungsteilers gestattet einen relativ einfachen Aufbau einer solchen Testeinrichtung, die es aber trotzdem zuläßt, die verwendeten Spektralfarben kontinuierlich einzustellen, so daß der Anomalquotient gemäß DIN 6160 ohne Schwierigkeiten ermittelt werden kann. Die Verwendung von Lumineszenzdioden eines jeweils engen Spektralbereiches, der zudem noch durch vorgesetzte Interferenz- oder Bandsperrfilter auf die gewünschte Wellenlänge eines monochromatischen Lichtes eingeschränkt werden kann, erlaubt eine kontinuierliche Regelung der Farbanteile bei dem Mischlicht. Allerdings könnte ein Anomaloskop dieser Art weit umfangreicher eingesetzt werden, wenn es möglich wäre, die Intensität beider Lichtarten zu erhöhen, ohne daß die Leistung der verwendeten Lumineszenzdioden gesteigert werden müßte.

Es ist deshalb die Aufgabe der Erfindung, das eingangs näher bezeichnete Anomaloskop weiter zu verbessern, ohne seine gegenüber dem sonstigen Stand der Technik erzielten Vorteile, insbesondere der einfachen Konstruktion und der sicheren Handhabung, aufzugeben. Besonders angestrebt wird dabei eine Erhöhung der Lichtintensität und ferner eine Verbesserung der Homogenität beider Lichtarten, um dem Probanden den Test zu erleichtern und fehlerhafte Aussagen einzuschränken.

Erfindungsgemäß wird die Aufgabe durch den kennzeichnenden Teil des Anspruches 1 gelöst. Ausgestaltungen der Erfindung enthalten die Ansprüche 2 bis 12.

Die Verwendung einer Ulbricht-Kugel ist in der Optik zwar bereits bekannt (Naumann/Schröder, Bauelemente der Optik, München/Wien 1983, S. 495). Sie wird dabei vor allem zur Lichtstrommessung im wesentlichen jeweils einer Lichtquelle benutzt. Im Gegensatz dazu stellt eine Ulbricht-Kugel in der erfindungsgemäßen Anordnung selbst eine originäre Lichtquelle dar, die zunächst zu beliebigen Beleuchtungszwecken gebraucht werden kann. Ihr Einsatz in einem Anomaloskop der beschriebenen Bauart gestattet nun tatsächlich, die Intensität beider verwendeter Lichtarten zu steigern. Abhängig von der Größe der verwendeten Ulbricht-Kugel können nunmehr relativ viele Lumineszenzdioden zur Erzeugung des benötigten Lichtstromes eingesetzt werden, entweder der gleichen Wellenlänge, wenn etwa ein gelbes Vergleichslicht produziert werden soll, oder verschiedener Wellenlänge, wenn die Erzeugung eines Mischlichtes erfolgen soll. Dabei ist die Verwendung nicht auf die Prüfung rot - grün beschränkt, vielmehr lassen sich nun gegebenenfalls Untersuchungen zum Farbensinn durchführen, die auch andere Spektralfarben einbeziehen, weil die entsprechenden Lumineszenzdioden in der gleichen Ulbricht-Kugel untergebracht werden können. Auch Ersatzdioden können vorgesehen sein, die erst dann zugeschaltet werden, wenn eine der Arbeitsdioden ausgefallen ist. Das Anomaloskop ist damit viel universeller einsetzbar und servicefreundlicher geworden.

Es versteht sich, daß das von einer Ulbricht-Kugel bereitgestellte Licht homogen in der Strahlungsebene ist; zum einen werden die Lichtstrahlen im Inneren der Ulbricht-Kugel vielfältig reflektiert, bevor sie aus der Meßöffnung austreten, zum anderen befindet sich bei einer Ulbricht-Kugel vor der Meßöffnung stets ein sog. Schatter, der verhindert, daß Licht aus einer Lichtquelle direkt austreten kann.

Die erreichbare hohe Lichtintensität läßt es nun auch unerheblich erscheinen, ob im Strahlengang Strahlungsteilerwürfel (Prismen) oder stattdessen halbdurchlässige Teilerspiegel als Funktionselemente verwendet werden; auch die letzteren erlauben trotz des unvermeidlich hohen Leistungsverlustes die Kontrolle der Aussagen des Probanden durch einen Mitbeobachter, für den ein gesonderter Strahlengang bereitstehen muß.

Weitere Vorteile und die näheren Einzelheiten der Erfindung werden nachfolgend anhand der Zeichnung an einem Ausführungsbeispiel näher erläutert. Es zeigen
- Fig. 1: eine erfindungsgemäße Anordnung in schematischer Darstellung,
- Fig. 2: einen Schnitt I-I durch das Reizfeld und
- Fig. 3 und 4: Schnitte II-II und III-III durch Teilfelder im Strahlengang eines erfindungsgemäßen Anomaloskops.

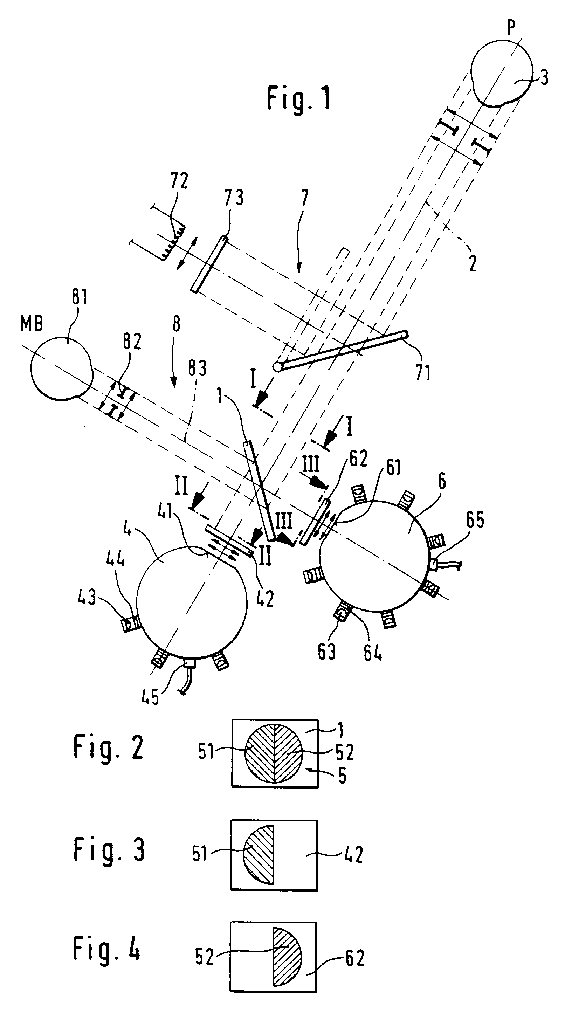

Ein erfindungsgemäßes Anomaloskop besteht zunächst gemäß Fig. 1 aus einem Strahlungsteiler 1, der hier als halbdurchlässiger Teilerspiegel ausgeführt ist und um 45° zur optischen Achse 2 des Gerätes angeordnet ist. Das Auge des Probanden, der in das in einem in der Zeichnung nicht dargestellten Gehäuse untergebrachte Anomaloskop blickt, ist mit 3 bezeichnet.

In der optischen Achse 2, jedoch auf der dem Auge 3 des Probanden gegenüberliegenden Seite des Strahlungsteilers 1 befindet sich eine erste Ulbricht-Kugel 4, aus deren Meßöffnung 41 eine erste Lichtart, hier ein gelbes Vergleichslicht, durch eine Blende 42 austritt, so daß ein erstes Teilfeld 51 eines Reizfeldes 5 (Fig. 2 und 3) entsteht, das ohne Umlenkung direkt in das Auge 3 gelangt.

Senkrecht zur optischen Achse 2 ist eine zweite Ulbricht-Kugel 6 angeordnet, aus deren Meßöffnung 61 eine zweite Lichtart, hier ein Mischlicht aus zwei Spektralfarben, durch eine Blende 62 austritt, so daß ein zweites Teilfeld 52 des Reizfeldes 5 (Fig. 2, 4) entsteht, das am Strahlungsteiler 1 reflektiert wird und dann ebenfalls in das Auge 3 gelangt.

In beiden Ulbricht-Kugeln 4 und 6 sind jeweils Lumineszenzdioden 43 bzw. 63 mit vorgesetzten Interferenzfiltern 44 bzw. 64 eingebaut. Die geringe Anzahl Lumineszenzdioden 43 soll versinnbildlichen, daß für die Erzeugung des gelben Vergleichslichtes weniger Lichtquellen erforderlich sind als für diejenige eines beispielsweise rot-grünen Mischlichtes, bei denen beispielsweise etwa die gleiche Anzahl roter und grüner Lumineszenzdioden 63 nötig sind. Elektrische kombinierte Energie- und Steuerleitungen 45 bzw. 65 versorgen die Dioden mit elektrischem Strom und regeln gleichzeitig ihre Leistungsabgabe.

Ein zweiter Strahlungsteiler 71 einer Einrichtung 7 zur Neutralbestimmung kann in den Strahlengang der optischen Achse 2 unter ca. 45° eingeschwenkt werden, um ein in einer Halogenlampe 72 erzeugtes weißes Licht - durch ein Filter 73 auf eine Farbtemperatur von 6.770 K normiert - in das Auge 3 zu spiegeln, so daß jeder Farbentest unter gleichen physiologischen Anfangsbedingungen erfolgen kann.

Schließlich erlaubt der Strahlungsteiler 1 die Kontrolle der gesamten Untersuchung, so daß falsche Angaben des Probanden korrigiert werden können. Dazu ist eine Kontrolleinrichtung 8 vorgesehen, bei der das Auge 81 einer Bedienungsperson über eine Blende 82 entlang einer optischen Achse 83, die senkrecht zur Achse 2 ist, auf das Reizfeld 5 am Strahlungsteiler 1 gerichtet ist.

## Patentansprüche

1. Anomaloskop zur Untersuchung des menschlichen Farbensinns mit einem ersten Strahlteiler (1) und mit zwei Lichtguellen (4, 6), die so angeordnet sind, daß ihr Licht jeweils unter etwa 45° auf verschiedenen Seiten der Strahlteilerebene auftrifft, von dieser zu einer für das Auge (3) eines Probanden vorgesehenen Position reflektiert bzw. transmittiert wird und diesem als ein aus zwei nebeneinander liegenden Teilfeldern (51, 52) bestehendes Reizfeld dargeboten werden kann, wobei die Richtung des transmittierten Lichts die optische Achse (2) des Anomaloskops festlegt und wobei das Licht einer Lichtquelle ein gelbes Vergleichslicht einer Wellenlänge und das Licht der anderen Lichtquelle ein Mischlicht aus zwei Spektralfarben ist, deren Anteile derart mischbar sind, daß bei Erhöhung des einen Anteils der jeweils andere in gleicher Weise erniedrigt wird, mit Mitteln zum Anzeigen der jeweiligen Lichtintensitäten sowie der Anteile der jeweiligen Spektralfarben und mit Mitteln zur Berechnung des Anomalquotienten,
dadurch gekennzeichnet,
daß mindestens eine der Lichtquellen (4, 6) aus einer Ulbrichtkugel besteht, in der sich mindestens eine Leuchtdiode (43, 63) befindet, deren Licht durch die Meßöffung (41, 61) der Kugel austritt.

2. Anomaloskop nach Anspruch 1, dadurch gekennzeichnet, daß die Meßöffnung (41; 61) durch eine Blende (42; 62) so abgedeckt ist, daß ein Teilfeld (51; 52) des Reizfeldes (5) aus einer der beiden Lichtarten gebildet wird, während das andere Teilfeld (51; 52) des Reizfeldes (5) durch die andere Lichtart in der gleichen Weise erzeugt wird.

3. Anomaloskop nach Anspruch 1, dadurch gekennzeichnet, daß die beiden Lichtarten in Lumineszenzdioden (43; 63) mit einem engen Spektralbereich erzeugt werden.

4. Anomaloskop nach Anspruch 1 und 3, dadurch gekennzeichnet, daß den Lumineszenzdioden (43; 63) ein oder mehrere Bandfilter oder Interferenzfilter (44; 64) vorgeschaltet sind.

5. Anomaloskop nach einem der Ansprüche 1 bis 4, dadurch gekennzeichet, daß auf der dem Probanden zugewandten Seite der Strahlungsteilerebene des Strahlungsteilers (1) eine Blende angeordnet ist.

6. Anomaloskop nach Anspruch 5, dadurch gekennzeichnet, daß in der optischen Achse (2) des Anomaloskops ein weiterer Strahlungsteiler (71) eingeschaltet ist, dessen Strahlungsteilerebene um etwa 45° zur optischen Achse (2) geneigt ist, und daß eine Halogenlampe (72) senkrecht zur optischen Achse (2) weißes Licht auf die Strahlungsteilerebene des Strahlungsteilers (71) projiziert.

7. Anomaloskop nach Anspruch 6, dadurch gekennzeichnet, daß in den Strahlengang zwischen der Halogenlampe (72) und der Strahlungsteilerebene des zweiten Strahlungsteilers (71) eine Blende (73) angeordnet ist.

8. Anomaloskop nach einem der Ansprüche 1 bis 7, dadurch gekennzeichnet, daß eine Kontrolleinrichtung (8) vorgesehen ist, mittels der das Auge (71) einer Bedienungsperson durch eine Blende (82) entlang einer optischen Achse (83), die senkrecht zur optischen Achse des Anomaloskops (2) ist, auf das Reizfeld (5) am Strahlungsteiler (1) gerichtet werden kann.

9. Anomaloskop nach einem der Ansprüche 1 bis 8, dadurch gekennzeichnet, daß das Anomaloskop in einem Gehäuse angeordnet ist.

10. Anomaloskop nach einem der Ansprüche 1 bis 9, dadurch gekennzeichnet das die erhaltenen Anomalquotientenwerte entsprechend der Rayleigh-Gleichung umgerechnet und digital angezeigt sind.

11. Anomaloskop nach einem der Ansprüche 1 bis 10, dadurch gekennzeichnet, daß die Intensitätssteuerung der Lichtquellen durch Pulsbreitenmodulation erfolgt und daß der Wechsel zwischen Ein- und Ausschalten mit einer Frequenz erfolgt, die deutlich über der Flimmerfusionsgrenze des menschlichen Auges liegt.

12. Anomaloskop nach Anspruch 11, dadurch gekennzeichnet, daß die Frequenz etwa 100 Hz beträgt.

## Claims

1. An anomaloscope for testing colour sense in humans, comprising a first beam splitter (1) and two light sources (4, 6) arranged in such a manner that the light therefrom strikes different sides of the beam splitter plane at approximately 45°, is reflected or transmitted from the said plane to a position provided for the eye (3) of a patient and can be presented to the patient as a stimulus field consisting of two adjacent sub-fields (51, 52), wherein the direction of the transmitted light determines the optical axis (2) of the anomaloscope and wherein the light from one light source is a yellow comparison light of one wavelength and the light from the other light source is a mixed light of two spectral colours, the proportions of which are miscible in such a manner that, when one proportion is increased, the other proportion is correspondingly decreased, comprising means for indicating the respective light intensities and the proportions of the respective spectral colours and comprising means for calculating the anomalous quotient, characterised in that at least one of the light sources (4, 6) comprises an Ulbricht sphere containing at least one light-emitting diode (43, 63), the light from which emerges through the measuring aperture (41, 61) of the sphere.

2. An anomaloscope according to claim 1, characterised in that the measuring aperture (41; 61) is covered by a stop (42; 62) in such a manner that one sub-field (51; 52) of the stimulus field (5) is formed from one of the two light types, while the other sub-field (51; 52) of the stimulus field (5) is generated by the other light type in the same manner.

3. An anomaloscope according to claim 1, characterised in that the two light types are generated in light-emitting diodes (43; 63) with a narrow spectral range.

4. An anomaloscope according to claims 1 and 3, characterised in that one or more band filters or interference filters (44; 64) are arranged upstream of the light-emitting diodes (43; 63).

5. An anomaloscope according to any one of claims 1 to 4, characterised in that a stop is arranged on the side of the beam splitter plane of the beam splitter (1) facing the patient.

6. An anomaloscope according to claim 5, characterised in that a further beam splitter (71) is arranged in the optical axis (2) of the anomaloscope and its beam splitter plane is at an angle of approximately 45° to the optical axis (2), and in that a halogen lamp (72) projects white light onto the beam splitter plane of the beam splitter (71) perpendicularly to the optical axis (2).

7. An anomaloscope according to claim 6, characterised in that a stop (73) is arranged in the beam path between the halogen lamp (72) and the beam splitter plane of the second beam splitter (71).

8. An anomaloscope according to any one of claims 1 to 7, characterised in that a monitoring device (8) is provided, by means of which the eye (71) of an operator can be directed through a stop (82), along an optical axis (83), which is perpendicular to the optical axis of the anomaloscope (2), and onto the stimulus field (5) on the beam splitter (1).

9. An anomaloscope according to any one of claims 1 to 8, characterised in that the anomaloscope is arranged in a housing.

10. An anomaloscope according to any one of claims 1 to 9, characterised in that the anomalous quotient values obtained are converted in accordance with the Rayleigh equation and displayed digitally.

11. An anomaloscope according to any one of claims 1 to 10, characterised in that the intensity of the light sources is controlled by pulse width modulation and in that the shift between switching on and switching off takes place at a frequency which is clearly above the flicker fusion threshold of the human eye.

12. An anomaloscope according to claim 11, characterised in that the frequency is approximately 100 Hz.

## Revendications

1. Anomaloscope destiné à l'examen du sens des couleurs chez l'homme, comportant une première lame séparatrice de faisceau (1) et deux sources lumineuses (4, 6) disposées de telle manière que leur lumière frappe respectivement les deux côtés du plan de la lame séparatrice sous un angle d'environ 45°, est réfléchie ou transmise à partir de celle-ci vers une position prévue pour l'oeil (3) d'une personne examinée et peut être présentée à celle-ci comme un champ stimulateur composé de deux champs partiels (51, 52) situés l'un à côté de l'autre,
dans lequel la direction de la lumière transmise détermine l'axe optique (2) de l'anomaloscope,
et dans lequel la lumière d'une source lumineuse est une lumière de référence jaune monochromatique, et la lumière de l'autre source lumineuse, un mélange de lumières de deux couleurs spectrales dont les parties composantes peuvent être mélangées de manière telle que l'augmentation d'une des parties composantes est accompagnée de la diminution similaire de l'autre partie composante,
comportant des moyens pour indiquer les intensités lumineuses ainsi que les quote-parts de chaque couleur spectrale et comportant des moyens pour calculer les quotients d'anomalie,
caractérisé en ce qu'au moins une des sources lumineuses (4, 6) est constituée par une sphère d'Ulbricht dans laquelle se trouve au moins une diode électroluminescente (43, 63), dont la lumière sort par l'ouverture de mesure (41, 61) de la sphère.

2. Anomaloscope selon la revendication 1, caractérisé en ce que l'ouverture de mesure (41, 61) est masquée par un diaphragme (42, 62) de manière à constituer un champ partiel (51, 52) du champ stimulateur (5) à partir d'une des deux sortes de lumière, alors que l'autre champ partiel (51, 52) du champ stimulateur (5) est produit de la même manière par l'autre sorte de lumière.

3. Anomaloscope selon la revendication 1, caractérisé en ce que les deux sortes de lumière sont produites dans des diodes électroluminescentes (43, 63) avec un domaine spectral étroit.

4. Anomaloscope selon les revendication 1 et 3, caractérisé en ce qu'un ou plusieurs filtres de bande ou filtres interférentiels sont placés devant les diodes électroluminescentes (43, 63).

5. Anomaloscope selon l'une des revendications 1 à 4, caractérisé en ce qu'un diaphragme est disposé du côté tourné vers la personne examinée du plan de la lame séparatrice de faisceau(1).

6. Anomaloscope selon la revendication 5, caractérisé en ce que, sur l'axe optique (2) de l'anomaloscope, est interposée une autre lame séparatrice de faisceau (71), dont le plan est incliné d'environ 45° par rapport à l'axe optique (2), et en ce qu'une lampe à halogène (72) projette perpendiculairement à l'axe optique (2) une lumière blanche sur le plan de la lame séparatrice de faisceau (71).

7. Anomaloscope selon la revendication 6, caractérisé en ce qu'un diaphragme (73) est disposé sur le trajet des rayons entre la lampe à halogène (72) et le plan de la deuxième lame séparatrice de faisceau (71).

8. Anomaloscope selon l'une des revendications 1 à 7, caractérisé en ce qu'il est prévu un dispositif de contrôle (8) au moyen duquel l'oeil (81) d'un opérateur peut être dirigé, à travers un diaphragme (82) situé le long d'un axe optique (83) perpendiculaire à l'axe optique de l'anomaloscope (2), vers le champ stimulateur (5) sur la lame séparatrice de faisceau.

9. Anomaloscope selon l'une des revendications 1 à 8, caractérisé en ce que l'anomaloscope est disposé dans un boîtier.

10. Anomaloscope selon l'une des revendications 1 à 9, caractérisé en ce que les valeurs des quotients d'anomalie obtenues sont calculées d'après l'équation de Rayleigh et affichées numériquement.

11. Anomaloscope selon l'une des revendications 1 à 10, caractérisé en ce que la commande d'intensité des sources lumineuses se fait par modulation en largeur d'impulsions et que l'alternance marche-arrêt se fait à une fréquence nettement supérieure à la fréquence de fusion du scintilement de l'oeil humain.

12. Anomaloscope selon la revendication 11, caractérisé en ce que la fréquence est d'environ 100 Hz.
